(19) Europäisches Patentamt

European Patent Office

Office européen des brevets

(11) **EP 1 428 523 A1**

(12) **EUROPEAN PATENT APPLICATION**

(43) Date of publication:
**16.06.2004 Bulletin 2004/25**

(51) Int Cl.⁷: $A61K\ 7/48$

(21) Application number: **03292955.6**

(22) Date of filing: **27.11.2003**

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority: **29.11.2002 JP 2002348360**
**20.12.2002 JP 2002370231**
**20.12.2002 JP 2002370238**

(71) Applicant: **Takasago International Corporation Tokyo-to 144-8721 (JP)**

(72) Inventors:
- **Kawada, Izumi, Takasago Int. Corp. Hiratsuka-shi Kanagawa 254-0073 (JP)**
- **Ishida, Misaki Amagasaki-shi Hyogo (JP)**

(74) Representative: **Uchida, Kenji et al S.A. Fedit-Loriot et Autres Conseils en Propriété Industrielle, 38, avenue Hoche 75008 Paris (FR)**

(54) **Skin cosmetics**

(57) Skin cosmetics which are excellent in moistness and persistency of moisture-keeping effect, show light sense of touch when used and have excellent periodical stability. In addition, there are provided skin cosmetics which do not cause stickiness after their use, are excellent in rough skin improving and preventing effects, wrinkle preventing and improving effects, dinginess improving affect and the like, and also have an effect in providing the skin with smoothness and liveliness. Also, in the case of skin cleansing compositions, they exert such effects that they can provide the skin with liveliness and moistness after washing and can effectively remove fishy and ammonia smells or the like filthy smell adhered to the hand, the skin and the like. A skin cosmetic characterized in that it contains (A) at least one substance selected from seed extracts and seed hull extracts of a plant belonging to the genus *Carya* of the family *Juglandaceae* in an amount of from 0.00005 to 2% by weight as solid matter, and (B) an additive agent.

**EP 1 428 523 A1**

**Description**

FIELD OF THE INVENTION

This invention relates to lotion, emulsion, cream, pack, hair tonic, skin cleansing composition and the like cosmetics for skin use.

BACKGROUND OF THE INVENTION

In general, human cuticle surface (the skin) is covered with sebaceous membrane and transpiration of moisture is appropriately inhibited thereby, so that insufficient moisture in the skin is apt to entail rough skin and the like and cause wrinkles. Thus, it is very important from the viewpoint of skin health to maintain skin moisture within a suitable range. However, since the sebaceous membrane is frequently removed by the use of a skin cleansing composition at the time of face washing or bathing, the skin moisture is apt to be removed temporarily. Because of this, lotion, emulsion, cream, essence and the like moisture keeping cosmetics are used after face washing or after bathing for the purpose of supplying and maintaining moisture in the skin. In general, glycerol, 1,3-butylene glycol, sorbitol and the like polyhydric alcohols, pyrrolidonecarboxylic acid salts and the like are blended as moisture keeping agents in moisture keeping cosmetics, but though these agents are excellent in moisture retaining property under high humidity, their moisture retaining property under low humidity is poor, their persistency of moisture keeping effect is not sufficient and, in some cases, they conversely accelerate rough skin by absorbing the skin moisture.

Accordingly, studies have been carried out in recent years on various moisture keeping components. For example, studies have been conducted on various substances, such as chitin, chitosan and derivatives thereof, protein hydrolysates, hyaluronic acid and the like acidic mucopolysaccharides, plant extracts and the like, as moisture keeping components having high water retaining ability under low humidity. However, when these moisture keeping components were blended in cosmetics, it caused an disadvantage of conversely resulting in unpleasant stickiness due to their high moisture keeping ability. In addition, in order to make up for these disadvantages, an ester oil, a hydrocarbon oil and the like various oily components and oily components containing unsaturated fatty acids and derivatives thereof have been formulated in various cosmetics, but periodical stability of the cosmetics prepared by using these oily components was not sufficient due to their periodical oxidation and deterioration. Accordingly, various oil soluble antioxidants have been used in skin cosmetics with the aim of attaining stabilization of the products and prevention from the oxidation of sebum after their application, but their periodical stability is not sufficient yet, and they do not have both of the effects to prevent and improve rough skin.

On the other hand, for the purpose of reinforcing the effects to prevent and improve rough skin, attempts have been made to formulate various anti-inflammatory agents, such as glycyrrhizic acid salts, glycyrrhetinic acid derivatives, azulene derivatives and the like, together with these components and vitamins having high moisture keeping effect (JP-A-6-32728, U.S. Patent No. 5,686,086, U.S. Patent No. 5,962,000).

However, it is the present situation that a skin cosmetic which can add sufficient moisture keeping effect and moisture to the skin and is also periodically stable while showing good used feeling cannot be obtained even by these methods. What is more, there are no reports on a cosmetic which simultaneously has the effect to prevent and improve generation of wrinkles due to aging, ultraviolet rays and the like, by preventing and improving rough skin, the effect to provide liveliness by softening the skin, the effect to improve dinginess of the skin, the effect to prevent aging of the skin and the like.

Also, in the case of body shampoo, cleansing cream and the like skin cleansing cosmetics which are used at the time of washing, there are problems regarding loss of moisture from the skin caused by washing and their stimulation to the skin. Because of this, concern has been directed toward the development of a skin cleansing composition which can add moistness and liveliness to the skin without losing excess moisture therefrom and can wash and smooth out the skin without spoiling the refreshed feeling.

In addition, it is important also as a function of the skin cleansing composition to remove not only stains but also unpleasant smells remained on hands, the skin and the like, such as ammonia and fishy smalls, or the like filthy smells, but in reality, it is the present situation to rely on masking by perfume and the like because it is unable to remove these smells efficiently. Furthermore, it is necessary to carry out excess washing for sufficiently removing stains and smells, but it causes problems such as chapping of the skin, so that it is the present situation that moistness and liveliness can hardly be retained on the skin after washing without using lotion, cream and the like moisture keeping cosmetics.

SUMMARY OF THE INVENTION

Under such situations, the invention contemplates solving the aforementioned problems and thereby providing periodically stable skin cosmetics which can add sufficient moisture keeping effect and moistness to the skin and also have good used feeling. Also, regarding skin cleansing compositions, it contemplates providing a skin cleansing composition which can effectively remove fishy and ammonia smells and the like filthy smells adhered to hands, the skin and the like.

In addition, it contemplates providing a skin cosmetic which shows light feeling during its use, does not cause stickiness after its use and is excellent in the rough skin-preventing and improving effects, the wrinkles-preventing and improving effects and the skin dinginess-improving effect, simultaneously having the effect to keep the skin smooth and young thereby providing liveliness and the like.

As a result of intensive studies carried out with the aim of solving the aforementioned problems, the present inventors have noticed that extracts of seeds and seed hulls of a plant belonging to the genus *Carya* of the family *Juglandaceae* can attain the aforementioned objects.

That is, it was found that the aforementioned objects of the invention can be attained by skin cosmetics which contain the aforementioned extract and an anti-inflammatory agent or oil soluble antioxidant at a specified ratio.

In addition, taking note of the function of the aforementioned extracts to remove ammonia and fishy smells and the like filthy smells and its effect to add liveliness and moistness to the skin after washing, it was found that the objects can be attained by a cleansing composition which contains a specified fatty acid soap at a predetermined ratio as the washing active ingredient and the aforementioned extract at a predetermined ratio, and also contains an amphoteric surface active agent and/or alkanolamide type nonionic surface active agent as occasion demands. The invention has been accomplished based on such knowledge.

Accordingly, the invention provides,

(1) a skin cosmetic which contains (A) at least one substance selected from seed extracts and seed hull extracts of a plant belonging to the genus *Carya* of the family *Juglandaceae* in an amount of from 0.00005 to 2% by weight as solid matter, and (B) an additive agent,

(2) the skin cosmetic described in the aforementioned item (1), wherein the additive agent (B) is an anti-inflammatory agent,

(3) the skin cosmetic described in the aforementioned item (2), wherein the anti-inflammatory agent is at least one compound selected from glycyrrhizic acid, glycyrrhetinic acid, azulene, allantoin and derivatives thereof,

(4) the skin cosmetic described in the aforementioned item (1), wherein the additive agent (B) is an oil soluble antioxidant,

(5) the skin cosmetic described in the aforementioned item (4), wherein the oil soluble antioxidant is at least one compound selected from vitamin E, vitamin A, derivatives thereof, and vitamin C derivatives,

(6) the skin cosmetic described in the aforementioned item (1), wherein the additive agent (B) is a fatty acid soap having from 8 to 20 carbon atoms,

(7) the skin cosmetic described in the aforementioned item (1), wherein the additive agent (B) is a fatty acid soap having from 8 to 20 carbon atoms, and an amphoteric surface active agent and/or an alkanolamide nonionic surface active agent,

(8) the skin cosmetic described in the aforementioned item (6) , wherein the skin cosmetic is a skin cleansing composition, and

(9) the skin cosmetic described in the aforementioned item (7), wherein the skin cosmetic is a skin cleansing composition.

DETAILED DESCRIPTION OF THE INVENTION

The skin cosmetics of the invention contain, as a component (A), at least one substance selected from seed extracts and seed hull extracts of a plant belonging to the genus *Carya* of the family *Juglandaceae.* Production method of the extract is not particularly limited, and the extract of interest can be obtained, for example, by subjecting seeds and/or seed hulls of a plant belonging to the genus *Carya* of the family *Juglandaceae*, illustratively a hickory (*C. tomentosa* (Poi Nutt)), pecan (*Carya illinoensis* (Wang) K. Koch) or the like, as such or after drying, to heating reflux together with one or two or more solvents selected from hydrocarbon compounds, esters, ketones, ethers, halogenated hydrocarbon compounds, alcohols and water, or by soaking them in such solvents . Preferred is an extract prepared by extracting seed hulls of pecan which belongs to the genus *Carya* of the family *Juglandaceae* with water and/or one or two or more of lower alcohols, and more preferred extract is their water extract.

The method shown below is an illustrative example of the extraction method (cf. JP-A-2000-72686).

Firstly, the seeds and/or seed hulls to be treated are pulverized as such or after drying and then subjected to extraction treatment using an extraction solvent in an amount of approximately from 5 to 100 times of their weight. The extraction temperature is optionally selected in response to the kind of extraction solvent to be used. For example, when the extraction solvent is water, the extraction temperature is generally from about 20 to about 120°C, preferably from about 90 to 110°C. Also, when the extraction solvent is a lower alcohol, it is generally from about 20 to about 50°C, preferably room temperature. Also, the extraction time may be approximately from 5 to 60 minutes when the extraction solvent is water, but a period of approximately from 24 to 96 hours is appropriate in the case of a lower alcohol.

After the extraction treatment, the extract is taken out by carrying out solid-liquid separation by filtration or the like means, the extraction solvent is evaporated in accordance with a general method and then a drying treatment is carried out as occasion demands, thereby obtaining the extract.

Amount of the aforementioned extract to be contained as the component (A) of the invention is selected within the range of from 0.00005 to 2% by weight, as solid matter based on the entire amount of the cosmetics. When this amount is less that 0.00005% by weight, persistency of the moisture keeping effect, rough skin-preventing and improving effect, wrinkles-preventing and improving effect, and the effect to make the skin smooth and thereby provide liveliness become weak. Also, when used in a skin cleansing composition, effective removal of ammonia and fishy smells and the like filthy smells cannot be made. When it exceeds 2% by weight on the other hand, not only the periodical stability becomes poof but also it is disadvantageous from the viewpoint of cost, and creaminess of foams becomes poor when used in a cleansing composition. From the above points of view, preferred amount of said component (A) to be contained is within the range of from 0.0001 to 1.5% by weight as solid matter, more preferably from 0.0005 to 1% by weight as solid matter.

In this connection, according to the invention, the extract of component (A) is formulated as the extract itself, or as its concentrated or dried preparation. Accordingly, when said extract contains an extraction solvent, the solid content is obtained as a calculated value by determining amount of the solvent using a gas chromatography or high performance liquid chromatography.

An additive agent is added to the skin cosmetics of the invention as the component (B).

Examples of the additive agent include an anti-inflammatory agent, an oil soluble antioxidant, a fatty acid soap having from 8 to 20 carbon atoms, an amphoteric surface active agent, an alkanolamide nonionic surface active agent and the like.

The anti-inflammatory agent is not particularly limited, and its useful examples include glycyrrhizic acid, glycyrrhetinic acid, azulene, allantoin, photosensitizer, ketoprofen, ibuprofen, camphor, thymol, piroxicam, salicylic acid, hydrocortisone, acetaminophen, aspirin, indometacin, bufexamac, saponin, oryzanol, ε-aminocaproic acid and salts thereof, ester derivatives and the like. According to the invention, among these agents, glycyrrhizic acid or a derivative thereof, glycyrrhetinic acid or a derivative thereof, azulene or a derivative thereof, and allantoin or a derivative thereof are suitably used.

These anti-inflammatory agents can be obtained for example in the following manner.

Glycyrrhizie acid can be obtained generally by treating an extract of licorice *(Glycyrrhiza glabra)* with a mineral acid or the like, and its salt is obtained by neutralizing it with potassium hydroxide, ammonia or the like alkali. Also, glycyrrhetinic acid is obtained when glycyrrhizic acid is decomposed using sulfuric acid or the like, and a derivative thereof can be obtained by allowing it to react with stearyl bromide or the like.

Regarding azulene, corresponding compounds are obtained from various essential oils; for example, guaiazulene is obtained from essential oil of *Guaiacum officinale* L. or guaiol. In addition, it can also be obtained by a synthesis method in which octalin is used as the starting material and, via a diketone and an unsaturated ketone, finally subjected to dehydrogenation (Plattner method) , or in which pyrimidine is used as the starting material and converted into a glutaconaldehyde derivative, and a condensation product of this with cyclopentadiene is heated (Ziegler process).

Allantoin is distributed in rootstocks of *Symphytum officinale* L., leaves of *Platanus orientalis* L. and the like, and it can be obtained by extracting from them but can also be obtained by a synthesis method in which bromine is allowed to act upon hydantoin and urea or it is condensed from two urea molecules and one glyoxylic acid molecule under compression and heating. In addition, its derivatives include allantoin chlorohydroxyaluminum, allantoin dihydroxyaluminum and the like.

As the anti-inflammatory agents, commercially available products may be used.

According to the invention, the aforementioned anti-inflammatory agents may be used alone or as a combination of two or more. Also, the content is selected within the range of from 0.005 to 10% by weight based on the total amount of the cosmetic composition. When this amount is less than 0.005% by weight, rough skin preventing and improving effects and wrinkle preventing effect become weak, and when it exceeds 10% by weight on the other hand, not only it causes stickiness and a problem regarding stability but also it is disadvantageous in terms of cost. From such point of view, the amount of said anti-inflammatory agent to be contained is preferably within the range of from 0.01 to 7% by weight, more preferably from 0.02 to 5% by weight.

According to the invention, it is desirable that blending weight ratio (A/B) of the aforementioned component (A) and anti-inflammatory agent (B) is within the range of preferably from 1/50 to 10/1, particularly preferably from 1/30 to 5/1, from the viewpoints of the persistency of moisture keeping effect, rough skin preventing effect, wrinkles preventing and improving effects, skin softening effect and the like.

Regarding the oil soluble antioxidant of the invention, there is no particular limitation, and its useful examples include α, β, γ or δ-tocopherol and the like vitamin E components, dl-α-tocopherol acetate and the like vitamin E derivatives, retinol, 3-dehydroretinol and the like vitamin A components, retinol acetate, retinol palmitate, retinal, retinoic acid or a salt thereof, ethyl retinoate, butyl retinoate and the like esters, retinyl phosphate or a salt thereof and the like vitamin A derivatives, liver oil extracted from fishes containing a large amount of vitamin A, ascorbic acid palmitate and the like vitamin C derivatives, butyl hydroxy anisole (BHA), dibutyl hydroxy toluene (BHT), eugenol, isoeugenol, propyl gallate and sorbic acid, as well as sesamin and sesame oil containing the same in a large amount, and flavonoids. Among them, vitamin E or a derivative thereof, vitamin A or a derivative thereof and a vitamin C derivative are preferably used.

According to the invention, the aforementioned oil soluble antioxidants may be used alone or as a combination of two or more. Also, the content is selected within the range of from 0.001 to 10% by weight based on the total amount of the cosmetic composition. When this amount is less than 0.001% by weight, rough skin preventing effect, wrinkles preventing and improving effects and dinginess improving effect do not become sufficient, and when it exceeds 10% by weight on the other hand, the sense of touch at the time of use or after use, such as poor spreading or generation of stickiness or the like, is worsened, and it may also cause a problem regarding periodical stability. The amount of said oil soluble antioxidant to be contained is preferably within the range of from 0.005 to 8% by weight, more preferably from 0.01 to 5% by weight.

According to the invention, it is desirable that blending weight ratio (A/B) of the aforementioned component (A) and oil soluble antioxidant (B) is within the range of preferably from 1/20 to 5/1, particularly preferably from 1/10 to 3/1, from the viewpoints of the rough skin preventing effect, wrinkles improving effect, effect to provide moistness and liveliness to the skin, dinginess improving effect and the like.

In case that the cosmetics of the invention are skin cleansing compositions, a fatty acid soap having from 8 to 20 carbon atoms is added as the component (B). The fatty acid soap having from 8 to 20 carbon atoms of the invention is an alkali salt of a fatty acid having from 8 to 20 carbon atoms and is the active washing ingredient. The fatty acid having from 8 to 20 carbon atoms is not particularly limited and can be used by optionally selecting it from saturated or unsaturated fatty acids having from 8 to 20 carbon atoms conventionally used as the material of fatty acid soaps. Its illustrative examples include caprylic acid, capric acid, lauric acid, myristic acid, palmitic acid, stearic acid, oleic acid, linoleic acid, coconut oil fatty acids, palm kernel oil fatty acids, sunflower oil fatty acids, hardened tallow fatty acids and the like. In addition, examples of the ion pair forming alkali include an alkali metal hydroxide, ammonia, an organic amine, a basic amino acid and the like, and their illustrative examples include lithium hydroxide, sodium hydroxide, potassium hydroxide, triethanolamine, lysine, arginine and the like, of which sodium, hydroxide and potassium hydroxide are preferred.

According to the invention, the aforementioned fatty acid soaps may be used alone or as a combination of two or more. Also, the content is selected within the range of from 5 to 80% by weight based on the total amount of the cosmetic composition. When this amount is less than 5% by weight, sufficient foaming ability cannot be obtained, and when it exceeds 80% by weight on the other hand, a strained feeling after washing becomes strong. When the foaming ability, strained feeling after washing and the like are taken into consideration, the content is within the range of preferably from 7 to 80% by weight, particularly preferably from 10 to 80% by weight.

According to the cleansing composition of the invention, in order to improve its foam quality, stability and the like, an amphoteric surface active agent and/or an alkanolamide nonionic surface active agent can be contained as the component (B), as occasion demands, in addition to the fatty acid soap having from 8 to 20 carbon atoms.

Examples of the amphoteric surface active agent include betaine alkyldimethylaminoacetate, betaine amidopropyldimethylaminoacetate, an alkylhydroxyethylmethyl sulfobetaine, an amidoamino acid salt, an alkyliminodicarboxylic acid salt, an alkyliminodiacetic acid salt, an alkylaminoethylglycine salt and the like, of which betaine alkyldimethylaminoacetate, betaine amidopropyldimethylaminoacetate, an amidoamino acid salt and an alkyliminodicarboxylic acid salt are preferred, and betaine amidopropyldimethylaminoacetate, an amidoamino acid salt and an alkyliminodicarboxylic acid salt are more preferred.

On the other hand, the alkanolamide nonionic surface active agent is prepared by the condensation reaction of a fatty acid with an alkanolamine or the aminolysis reaction of oil and fat with an alkanolamine. Examples of the fatty acid which forms said compound include caprylic acid, capric acid, lauric acid, myristic acid, palmitic acid, stearic acid, oleic acid, linoleic acid, coconut oil fatty acids, palm kernel oil fatty acids, sunflower oil fatty acids, hardened tallow fatty acids and the like, and examples of the alkanolamine include monoethanolamine, diethanolamine, monoisopropanolamine and the like. Examples of the oil and fat which form said compound include coconut oil, palm kernel oil, sunflower oil, hardened beef tallow and the like. In addition, a 1:1 type or 1:2 type is prepared depending on the reaction condition, but preferred is a 1:1 type.

According to the invention, the aforementioned amphoteric surface active agents may be used alone or as a combination of two or more, and the aforementioned alkanolamide nonionic surface active agents may be used alone or as a combination of two or more. Also, one or more amphoteric surface active agents and one or more alkanolamide nonionic surface active agents may be used in combination. In addition, it is desirable that the total, content of the amphoteric surface active agents and/or alkanolamide nonionic surface active agents is within the range of from 0.1 to 10% by weight, based on the total amount of the cosmetic composition. The amount within the above range is desirable, because effect of the surface active agent is sufficiently exerted and the sense of touch after completion of washing becomes good without sliminess. More preferred amount of said component is within the range of from 0.5 to 8% by weight.

As occasion demands, various components generally used in the conventional skin cosmetics can be formulated in the skin cosmetics of the invention. For example, it can be blended with lower alcohols, polyhydric alcohols, hydrocarbon oils, natural oils and fats, ester oils, waxes, silicone derivatives, oily bases, other surface active agents, alkylamine or amidoamine hydrochlorides or acetates, inorganic or organic powders, various pigments, natural dyestuffs, water-soluble polymer compounds, organic or inorganic salts, pH adjusting agents, germicides, antiseptic and antimicrobial agents, chelating agents, other antioxidants, ultraviolet ray absorbents, other animal- and plant-derived natural extracts, perfumes, water and the like.

In addition, when an anti-inflammatory agent is used as the component B of the skin cosmetics of the invention, an oil soluble antioxidant, a fatty acid soap having from 8 to 20 carbon atoms, an amphoteric surface active agent and an alkanolamide nonionic surface active agent as other components B can be blended as occasion demands, similar to the case of the aforementioned various components. When an oil soluble antioxidant is used as the component B, an anti-inflammatory agent, a fatty acid soap having from 8 to 20 carbon atoms, an amphoteric surface active agent and an alkanolamide nonionic surface active agent as other components B can be blended as occasion demands, similar to the case of the aforementioned various components. When a fatty acid soap having from 8 to 20 carbon atoms, an amphoteric surface active agent and an alkanolamide nonionic surface active agent are used as the component B, an anti-inflammatory agent and an oil soluble antioxidant as other components B can be blended as occasion demands, similar to the case of the aforementioned various components.

Examples of the aforementioned lower alcohols include ethanol, isopropyl alcohol and the like, and examples of the polyhydric alcohols include propylene glycol, dipropylene glycol, glycerol, diglycerol, 2,3-butylene glycol, polyethylene glycol, sorbitol, maltitol and the like.

Examples of the hydrocarbon oils include liquid paraffin, liquid isoparaffin, squalane, vaseline, solid paraffin and the like, examples of the natural oils and fats include beef tallow, lard, fish oil and the like, and examples of the ester oils include glyceryl tri-2-ethylhexanoate or the like synthetic triglyceride, isopropyl myristate, isopropyl palmitate, cetyl palmitate, ethyl oleate, oleyl oleate, octyldodecyl myristate and the like,

Examples of the waxes include beeswax, carnauba wax and the like, examples of the silicone derivatives include straight chain or cyclic dimethyl polysiloxane, polyether-denatured dimethyl polysiloxane, amino-denatured dimethyl polysiloxane and the like, and examples of the oily bases include ceramide, cholesterol, a protein derivative, lanolin, a lanolin derivative, lecithin and the like.

Examples of the other surface active agents include polyoxyethylene alkyl ether, polyethylene glycol fatty acid ester, polyoxypropylene alkyl ether, polypropylene glycol fatty acid ester, sorbitan fatty acid ester, polyoxyethylene sorbitan fatty acid ester, polyoxyethylene hydrogenated castor oil, polyglycerol fatty acid ester, polyoxyethyleneglycerol fatty acid ester, glycerol mono-fatty acid ester, alkyl polyglucoside, polyoxyethylene polyoxypropylene block polymer and the like nonionic surface active agents; other soaps, alkyl sulfuric acid ester salt, alkyl ether sulfuric acid ester salt, $\alpha$-olefin sulfonic acid salt, acyl methyltaurine salt, acyl glutamic acid salt, acyl glycine salt, acyl sarcosine salt, acyl isethionic acid salt, alkyl ether carboxylic acid salt, amide ether sulfuric acid ester salt, alkyl phosphoric acid ester salt and the like anionic surface active agents; alkyl amine oxide, alkyl dimethylamine oxide, polyoxyethylene alkyl amine oxide and the like semi-polar surface active agents; and alkyl trimethylammonium chloride, dialkyl dimethylammonium chloride and the like cationic surface active agents.

Examples of the inorganic or organic powders include talc, kaolin, sericite, mica, vermiculite, magnesium carbonate, calcium carbonate, diatomaceous earth, magnesium silicate, calcium silicate, aluminum silicate, barium silicate, strontium silicate, barium sulfate, tungstic acid metal salt, silica, zeolite, hydroxyapatite, boron nitride, ceramics powder and the like inorganic powders ; and crystalline cellulose, polyethylene powder, polyethylene tetrafluoride powder and the like organic powders.

Also, examples of the various pigments include titanium oxide, zinc oxide, red iron oxide (rouge), Chinese yellow, carbon black, cobalt violet, chromium oxide, ultramarine and the like inorganic pigments; titanium oxide-coated mica, fish scale foil, titanium oxide blue-coated mica and the like pearl pigments; aluminum powder, copper powder and the like metal powder pigments; and Red No. 201, Orange No. 204, Yellow No. 205, Blue No. 404 and the like organic pigments.

In addition, examples of. the natural dyestuffs include Red No . 3, Red No. 106, Red No. 227, Yellow No. 4, Yellow No. 5, Blue No. 1 and the like zirconium, chlorophyll, $\beta$-carotene and the like, examples of the water-soluble polymer compounds include alginic acid, carboxyvinyl polymer, carboxymethylcellulose, hydroxypropylmethylcellulose, hydroxyethyl cellulose, xanthan gum, hyaluronic acid and the like, examples of the inorganic or organic salts include magnesium sulfate, sodium chloride, sodium citrate, sodium pyrrolidonecarboxylate, malic acid salt and the like, and examples of the pH adjusting agents include acids and alkalis.

The skin cosmetics of the invention are suitably used as lotion, milky lotion, emulsion, essence, cream, gel, pack cosmetics, mask cosmetics, rouge cosmetics, make up cosmetics, nail cosmetics, shaving foam, hair cosmetics such as hair tonic, hair rinse, hair spray, mousse and the like, bathing agent, deodorant cosmetics, sweat preventing agent, skin cleansing compositions such as soap, cleansing lotion, cleansing cream, cleansing foam, body shampoo, hand soap, shampoo and the like, and shampoo for animal use.

Next, the invention is described further illustratively based on examples, but the invention is not restricted by these examples.

Production Example 1 Production of pecan seed hull extract A-1

Hull-attached seeds of pecan belonging to the genus *Carya* of the family *Juglandaceae* (pecan nuts) were crushed as such, and 100 g of the crushed material was mixed with 500 ml of 50% by weight ethanol aqueous solution and allowed to stand at room temperature for 48 hours to obtain an extract. By evaporating the solvent from this extract under a reduced pressure, the pecan seed hull extract A-1 of interest (brown powder 7.2 g) was obtained.

Production Example 2 Production of pecan seed hull extract A-2

A 100 g portion of a pecan seed hull crushed material obtained in the same manner as in Production Example 1 was mixed with 500 ml of distilled water and heated at 110°C under reflux for 10 minutes to obtain an extract. By evaporating the solvent from this extract under a reduced pressure, the pecan seed hull extract A-2 of interest (light brown powder 6.5 g) was obtained.

Comparative Production Example 1 Production of walnut seed hull extract A-3

A 100 g portion of a seed hull crushed material of walnut belonging to the family *Juglandaceae* was mixed with 500 ml of distilled water and heated at 110°C under reflux for 10 minutes to obtain an extract. By evaporating the solvent from this extract under a reduced pressure, a walnut seed hull extract A-3 (brown powder 2.4 g) was obtained.

Inventive examples 1 to 3 and Comparative Examples 1 to 4

Skin cosmetics as transparent or semi-transparent lotions having the compositions shown in Table 5 were prepared and their performance was evaluated by the following methods. The results are shown in Table 5 (in Table 5, the adding amount indicates % by weight, the same shall apply hereinafter). In this case, the 4 components shown in the following Table 1 were used as common adding components.

Table 1

|   | Components | Adding amount (% by weight) |
|---|---|---|
| 1 | Sodium citrate dihydride | 0.3 |
| 2 | Ethanol | 3.0 |
| 3 | Methyl paraben | 0.1 |
| 4 | Phenoxyethyl alcohol | 0.2 |
| Total | | 3.6 |

(1) The sense of touch when used

Using a panel of 20 females (22 to 37 years old), the sense of touch when the skin cosmetics were used after face washing was judged as follows, and by obtaining average value of the 20 members, an average value of 1.5 points or more was evaluated as a cosmetic having a light sense of touch when used.

Two points: good spreading when used and felt no scratch but light sense of touch.

One point: slightly poor spreading when used and felt slightly scratching.

Zero point: poor spreading when used and felt heavy scratching.

(2) Stickiness after use

Using a panel of 20 females (22 to 37 years old), the sense of touch to the skin 10 minutes after the use of the skin cosmetics after face washing was judged as follows, and by obtaining average value of the 20 members, an average value of 1.5 points or more was evaluated as a cosmetic having no stickiness after its use.
Two points: felt no stickiness to the skin.
One point: felt a slight stickiness to the skin.
Zero point: felt sticky to the skin.

(3) Persistency of moisture keeping effect

Using a panel of 20 females (22 to 37 years old), the moistness of the skin 2 hours after the use of the skin cosmetics after face washing was judged as follows, and by obtaining average value of the 20 members, an average value of 1.5 points or more was evaluated as a cosmetic having good persistence of moisture keeping affect.
Two points: felt that the skin was sufficiently moist not changing from just after use.
One point: felt that moistness of the skin was slightly insufficient in comparison with just after use.
Zero point: felt that moistness of the skin was insufficient in comparison with just after use.

(4) Rough skin improving effect

Using a panel of 10 females having rough skin (25 to 35 years old), conditions of the skin when the skin cosmetics were used twice a day continuously for 2 weeks were judged as follows, and by obtaining average value of the 10 members, an average value of 1.5 points or more was evaluated as a cosmetic having rough skin improving effect.
Two points: felt that the rough skin was evidently healed.
One point: felt that the rough skin was slightly healed.
Zero point: felt that there was no rough skin improving effect.

(5) Wrinkle improving effect

Using a panel of 10 females (31 to 45 years old), conditions of the skin when the skin cosmetics were applied to the corner of the eye twice a day continuously for 2 weeks were judged by sensorial test as follows, and by obtaining average value of the 10 members, an average value of 1.5 points or more was evaluated as a cosmetic having excellent wrinkle improving effect.
Two points: felt that wrinkles became evidently unconspicuous.
One point: felt that wrinkles became slightly unconspicuous.
Zero point: felt that there was no wrinkle improving effect.

(6) Smoothness of the skin

Using a panel of 20 females (22 to 37 years old), conditions of the skin when the skin cosmetics were used twice a day continuously for 2 weeks were judged by sensorial test as follows, and by obtaining average value of the 20 members, an average value of 1.5 points or more was evaluated as a cosmetic having an effect to provide the skin with liveliness.
Two points: felt that the skin evidently has liveliness and moistness and is smooth.
One point: felt that the skin is not so smooth.
Zero point: felt that the skin has no liveliness and moistness and is not smooth.

(7) Periodical stability

Each of the cosmetics was sealed in a transparent glass container and stored at 0°C, 25°C or 40°C for 3 months, and its appearance was observed and evaluated by the following three steps.

O: stability is good (no changes in appearance at each temperature).
Δ: stability is slightly poor (lees and precipitate are slightly formed or a change of color is generated in some degree at any of the temperatures).
X: stability is poor (lees and precipitate are formed or separated, or a change of color is considerable at any of the temperatures).

According to Inventive Examples 1 to 3, each of the lotions which used the components of the invention is light in the sense of touch when used, not sticky after use and excellent in the persistency of moisture keeping effect, has superior rough skin improving effect and wrinkle improving effect, can make the skin smooth and is also excellent in periodical stability. On the other hand, sufficient performance was not obtained in Comparative Examples 1 to 4. That is, the persistency of moisture keeping effect, rough skin improving effect and wrinkle improving effect became weak and smoothness of the skin also became poor in Comparative Example 1 because of the absence of the component (A), and the rough skin improving effect and wrinkle improving effect became weak in Comparative Example 2 because of the absence of the component (B). In addition, the rough skin improving effect and wrinkle improving effect became weak and smoothness of the skin also became poor in Comparative Examples 3 and 4, because the walnut seed hull extract and hyaluronic acid as a high moisture keeping component were blended instead of the component (A).

Inventive Examples 4 to 6

Skin cosmetics as oil-in-water type emulsions shown in Table 6 were prepared and their evaluation was carried out by the same methods as in Inventive Examples 1 to 3. In this case, however, the 11 components shown in the following Table 2 were used as common adding components. The results are shown in Table 6 (in Table 6, the adding amount indicates % by weight, the same shall apply hereinafter).

Table 2

|  |  | Components | Adding amount (% by weight) |
|---|---|---|---|
|  | 1 | Xanthan gum ("Echo Gum T", mfd. by Dainippon Pharmaceutical) | 0.1 |
|  | 2 | Carboxyvinyl polymer (CaxboPole 940", mfd. by BF-GoodRich) | 0.12 |
|  | 3 | L-Arginine | 0.1 |
|  | 4 | Ethanol | 5 |
|  | 5 | Purified sunflower oil | 5 |
|  | 6 | Squalane | 3 |
|  | 7 | Octyldodecyl myristate | 2 |
|  | 8 | Polyoxyethylene stearyl ether (20 E.O.) | 0.7 |
|  | 9 | Methyl paraben | 0.1 |
|  | 10 | Butyl paraben | 0.05 |
|  | 11 | Phenoxyethyl alcohol | 0.2 |
|  | Total |  | 16.37 |

According to Inventive Examples 4 to 6, each of the emulsions of the invention is light in the sense of touch when used, not sticky after use and excellent in the persistency of moisture keeping effect, has superior rough skin improving effect and wrinkle improving effect, can make the skin smooth and is also excellent in periodical stability.

Inventive Examples 7 and 8

Skin cosmetics as oil-in-water type creams shown in Table 6 were prepared and their performance was evaluated by the same methods as in Inventive Examples 1 to 3, except that the periodical stability (7) was evaluated by the following method. In this case, however, the 8 components shown in the following Table 3 were used as common adding components. The results are shown in Table 6.

Table 3

|  |  | Components | Adding amount (% by weight) |
|---|---|---|---|
|  | 1 | Purified olive oil | 8 |
|  | 2 | Squalane | 3 |
|  | 3 | Octyldodecyl myristate | 2 |

Table 3 (continued)

|   | Components | Adding amount (% by weight) |
|---|---|---|
| 4 | Dimethyl polysiloxane (100 CS) | 1 |
| 5 | Purified beeswax | 3 |
| 6 | Methyl paraben | 0.2 |
| 7 | Propyl paraben | 0.1 |
| 8 | Butyl paraben | 0.1 |
| Total | | 17.4 |

(7) Periodical stability

Conditions when each of the cosmetics was sealed in a transparent glass container and stored at -5°C, 25°C or 45°C for 1 month were examined and evaluated by the following three steps.

O: stability is good (no changes in appearance and no formation of small cluster and crystal and the like).
Δ: stability is slightly poor (precipitate is slightly formed or separation is slightly observed, or small cluster and crystal and bigger cluster are slightly formed) .
X: stability is poor (precipitate is evidently formed or separated, and small cluster and crystal and bigger cluster are formed).

According to Inventive Examples 7 and 8, each of the creams of the invention is light in the sense of touch when used, not sticky after use and excellent in the persistency of moisture keeping effect, has superior rough skin improving effect and wrinkle improving effect, can make the skin smooth and is also excellent in periodical stability.

Inventive Examples 9 and 10

Skin cosmetics as water-in-oil type creams shown in Table 6 were prepared and their evaluation was carried out by the same methods as in Inventive Examples 7 and 8. In this case, however, the 9 components shown in the following Table 4 were used as common adding components. The results are shown in Table 6.

Table 4

|   | Components | Adding amount (% by weight) |
|---|---|---|
| 1 | Purified jojoba oil | 3 |
| 2 | Purified sunflower oil | 6 |
| 3 | Dimethyl polysiloxane (100 CS) | 1 |
| 4 | Squalane | 4 |
| 5 | Octyldodecyl myristate | 5 |
| 6 | Methyl paraben | 0.2 |
| 7 | Propyl paraben | 0.1 |
| 8 | Butyl paraben | 0.1 |
| 9 | Magnesium sulfate (heptahydrate) | 0.5 |
| Total | | 19.9 |

According to Inventive Examples 9 and 10, each of the creams of the invention is light in the sense of touch when used, not sticky after use and excellent in the persistency of moisture keeping effect, has superior rough skin improving effect and wrinkle improving effect, can make the skin smooth and is also excellent in periodical stability.

Table 5

| | | | Inventive Examples | | | Comparative Examples | | | |
|---|---|---|---|---|---|---|---|---|---|
| | | | 1 | 2 | 3 | 1 | 2 | 3 | 4 |
| Compositions of skin cosmetics | | | | | | | | | |
| | (A) | Pecan seed hull extract A-1 | 0.1 | - | - | - | - | - | - |
| | | Pecan seed hull extract A-2 | - | 0.2 | 0.5 | - | 0.2 | - | - |
| | (B) | Monoammonium glycryrrhizate | 0.1 | - | - | 0.1 | - | - | - |
| | | Dipotassium glycyrrhizate | - | - | 0.5 | - | - | 0.5 | - |
| | | Allantoin | - | 0.2 | - | - | - | - | 0.2 |
| Others | | | | | | | | | |
| | Na hyaluronate [note 1] | | - | 0.1 | - | - | 0.1 | - | 0.1 |
| | Walnut seed hull extract A-3 | | - | - | - | - | - | 0.2 | - |
| | Glycerol | | - | - | 2 | - | - | 2 | - |
| | Dipropylene glycol | | - | - | 3 | - | - | 3 | - |
| | Polyethylene glycol 1540 | | - | - | 2 | - | - | 2 | - |
| | Polyoxyethylene sorbitan monooleate (20 E.O.) | | - | - | 0.25 | - | - | 0.25 | |
| | Sodium pyrrolidonecarboxylate (50% aqueous solution) | | - | - | 0.3 | - | - | 0.3 | - |
| | Citric acid monohydrate | | 0.1 | 0.1 | 0.03 | 0.1 | 0.1 | 0.03 | 0.1 |
| | Oleyl alcohol | | - | - | 0.05 | - | - | 0.05 | - |
| | Perfume | | - | - | 0.05 | - | - | 0.05 | - |
| | Common additive components | | 3.6 | | | 3.6 | | | |
| Purified water | | | balance | | | balance | | | |
| Performance | | | | | | | | | |
| | The sense of touch when used | | 1.9 | 1.9 | 1.8 | 1.8 | 1.8 | 1.7 | 1.7 |
| | Stickiness after use | | 1.8 | 1.8 | 1.8 | 1.6 | 1.7 | 1.7 | 1.6 |
| | Persistency of moisture keeping effect | | 1.5 | 1.6 | 1.6 | 1.2 | 1.6 | 1.3 | 1.3 |
| | Rough skin improving effect | | 1.5 | 1.5 | 1.5 | 1.2 | 1.0 | 1.4 | 1.1 |
| | Wrinkle improving effect | | 1.5 | 1.5 | 1.6 | 0.6 | 1.3 | 1.1 | 0.8 |
| | Smoothness of the skin | | 1.6 | 1.7 | 1.6 | 1.3 | 1.6 | 1.1 | 1.1 |
| | Periodical stability | | O | O | O | O | O | O | O |

Note 1; sodium hyaluronate FCH-200 (mfd. by Kibun Food Chemifa)

## Table 6

| Compositions of skin cosmetics | | | Inventive Examples | | | | | | |
|---|---|---|---|---|---|---|---|---|---|
| | | | 4 | 5 | 6 | 7 | 8 | 9 | 10 |
| (A) | Pecan seed hull extract A-1 | | 0.1 | – | – | 0.2 | – | 0.5 | – |
| | Pecan seed hull extract A-2 | | – | 0.3 | 0.02 | – | 0.5 | – | 0.3 |
| (B) | Dipotassium glycyrrhizate | | 0.1 | – | – | – | – | – | – |
| | Stearyl glycyrrhizate | | – | – | 0.5 | 0.2 | – | 0.1 | 0.3 |
| | Guaiazulene | | – | – | – | – | 0.1 | – | 0.1 |
| | Allantoin | | – | 0.5 | – | – | 0.05 | 0.1 | – |
| Others | | | | | | | | | |
| Glycerol | | | 2 | 2 | 2 | 2 | 2 | 2 | 3 |
| Dipropylene glycol | | | 2 | 3 | 3 | 3 | 3 | – | – |
| Polyethylene glycol 1540 | | | 2 | – | – | – | – | – | – |
| Polyethylene glycol monostearate (1540) | | | 0.5 | 0.5 | 0.5 | – | – | – | – |
| Polyethylene glycol monostearate (4000) | | | – | – | – | 1 | 1 | – | – |
| Polyoxyethylene sorbitan monostearate (20 E.O.) | | | – | – | – | 1 | 1 | – | – |
| Glycerol monooleate | | | – | – | – | – | – | 1.5 | 1.5 |
| Diglycerol monooleate | | | – | – | – | – | – | 0.2 | 0.2 |
| Glycerol monostearate | | | 1 | 1 | 1 | 2 | 2 | – | – |
| Cetanol | | | 2 | – | – | 3 | – | – | 3 |
| Behenyl alcohol | | | – | 1 | 1 | – | 3 | 2 | – |
| Decamethylcyclopentane siloxane | | | – | – | – | 3 | – | – | – |
| Perfume | | | – | – | 0.05 | – | – | – | – |
| dl-α-Tocopherol acetate | | | – | – | – | 0.05 | 0.05 | 0.1 | 0.1 |
| Common additive components | | | 16.37 | | | 17.40 | | 19.9 | |
| Purified water | | | balance | | | balance | | balance | |
| Performance | | | | | | | | | |
| The sense of touch when used | | | 1.9 | 1.8 | 1.8 | 1.8 | 1.8 | 1.7 | 1.7 |
| Stickiness after use | | | 1.8 | 1.8 | 1.9 | 1.7 | 1.8 | 1.7 | 1.6 |
| Persistency of moisture keeping effect | | | 1.6 | 1.6 | 1.6 | 1.8 | 1.9 | 1.9 | 2.0 |
| Rough skin improving effect | | | 1.6 | 1.6 | 1.6 | 1.9 | 1.9 | 1.9 | 2.0 |
| Wrinkle improving effect | | | 1.6 | 1.7 | 1.6 | 1.9 | 2.0 | 2.0 | 1.9 |
| Smoothness of the skin | | | 1.6 | 1.6 | 1.6 | 1.9 | 1.8 | 1.8 | 1.8 |
| Periodical stability | | | O | O | O | O | O | O | O |

Inventive examples 11 to 13 and Comparative Examples 5 to 8

Skin cosmetics as transparent or semi-transparent lotions having the compositions shown in Table 7 were prepared and their performance was evaluated by the following methods. The results are shown in Table 7 (in Table 7, the adding amount is shown by % by weight, the same shall apply hereinafter). In this case, the 4 components shown in the aforementioned Table 1 were used as common adding components.

(1) The sense of touch when used

Using a panel of 20 females (22 to 37 years old), the sense of touch when the skin cosmetics were used after face washing was judged as follows, and by obtaining average value of the 20 members, an average value of 1.5 points or more was evaluated as a cosmetic having a light sense of touch when used.

Two points: good spreading when used and felt no scratch but light sense of touch.

One point: slightly poor spreading when used and felt slightly scratching.

Zero point: poor spreading when used and felt heavy scratching.

(2) Stickiness after use

Using a panel of 20 females (22 to 37 years old), the sense of touch to the skin 10 minutes after the use of the skin cosmetics after face washing was judged as follows, and by obtaining average value of the 20 members, an average value of 1.5 points or more was evaluated as a cosmetic having no stickiness after its use.

Two points: felt no stickiness to the skin.

One point: felt a slight stickiness to the skin.

Zero point: felt sticky to the skin.

(3) Rough skin improving effect

Using a panel of 10 females who are apt to cause rough skin (23 to 35 years old), conditions of the skin when the skin cosmetics were used twice a day continuously for 2 weeks were judged as follows, and by obtaining average value of the 10 members, an average value of 1.5 points or more was evaluated as a cosmetic having rough skin improving effect.

Two points: rough skin was not formed.

One point: felt that the rough skin was slightly formed.

Zero point: felt that there was no rough skin improving effect.

(4) Wrinkle improving effect

Using a panel of 10 females (31 to 45 years old), conditions of the skin when the skin cosmetics were applied to the corner of the eye twice a day continuously for 2 weeks were judged by sensorial test as follows, and by obtaining average value of the 10 members, an average value of 1.5 points or more was evaluated as a cosmetic having excellent wrinkle improving effect.

Two points: felt that wrinkles became evidently unconspicuous.

One point: felt that wrinkles became slightly unconspicuous.

Zero point: felt that there was no wrinkle improving effect.

(5) Effect to provide the skin with moistness and liveliness

Using a panel of 20 females (22 to 37 years old), conditions of the skin when the skin cosmetics were used twice a day continuously for 2 weeks were judged by sensorial test as follows, and by obtaining average value of the 20 members, an average value of 1.5 points or more was evaluated as a cosmetic having an effect to provide the skin with moistness and liveliness.

Two points: felt that the skin evidently gained moistness and liveliness.

One point: felt that the skin slightly gained moistness and liveliness.

Zero point: felt that the skin gained no moistness and liveliness.

(6) Skin dinginess improving effect

Using a panel of 20 females (22 to 37 years old), conditions of the skin when the skin cosmetics were used twice a day continuously for 2 weeks were judged by sensorial test as follows, and by obtaining average value of the 20 members, an average value of 1.5 points or more was evaluated as a cosmetic having excellent skin dinginess improving effect.

Two points: felt that dinginess became evidently unconspicuous.

One point: felt that dinginess became slightly unconspicuous.

Zero point: felt that there was no dinginess improving effect.

(7) Periodical stability

Each of the cosmetics was sealed in a transparent glass container and stored at 0°C, 25°C or 40°C for 3 months, and its appearance was observed and evaluated by the following three steps.

O: stability is good (no changes in appearance at each temperature).
∆: stability is slightly poor (lees and precipitate are slightly formed or a change of color is generated in some degree at any of the temperatures).
X: stability is poor (lees and precipitate are formed or separated, or a change of color is considerable at any of the temperatures).

According to Inventive Examples 11 to 13, each of the lotions which used the components of the invention is light in the sense of touch when used and not sticky after use, has superior rough skin improving effect and wrinkle improving effect, can provide the skin with moistness and liveliness, has excellent dinginess improving effect and is also excellent in periodical stability. On the other hand, sufficient performance was not obtained in Comparative Examples 5 to 8. That is, the rough skin improving effect, wrinkle improving effect, effect to provide the skin with moistness and liveliness and dinginess improving effect became weak in Comparative Example 5 because of the absence of the component (A), and the rough skin improving effect, wrinkle improving effect and dinginess improving effect became weak in Comparative Example 6 because of the absence of the component (B). In addition, the stability became poor and the wrinkle improving effect, effect to provide the skin with moistness and liveliness and dinginess improving effect became weak in Comparative Example 7 and Comparative Example 8, because the green tea catechin or walnut seed hull extract were blended instead of the component (A)

Inventive Examples 14 to 16

Skin cosmetics as oil-in-water type emulsions shown in Table 8 were prepared and their evaluation was carried out by the same methods as in Inventive Examples 11 to 13. In this case, however, the 11 components shown in the aforementioned Table 2 were used as common adding components. The results are shown in Table 8 (in Table 8, the adding amount indicates % by weight, the same shall apply hereinafter).
According to Inventive Examples 14 to 16, each of the emulsions of the invention is light in the sense of touch when used and not sticky after use, has superior rough skin improving effect and wrinkle improving effect, can provide the skin with moistness and liveliness, has excellent dinginess improving effect and is also excellent in periodical stability.

Inventive Examples 17 and 18

Skin cosmetics as oil-in-water type creams shown in Table 8 were prepared and their performance was evaluated by the same methods as in Inventive Examples 11 to 13, except that the periodical stability (7) was evaluated by the following method. In this case, however, the 8 components shown in the aforementioned Table 3 were used as common adding components, The results are shown in Table B.

(7) Periodical stability

Conditions when each of the cosmetics was sealed in a transparent glass container and stored at -5°C, 25°C or 45°C for 1 month were examined and evaluated by the following three steps.

O: stability is good (no changes in appearance and no formation of small cluster and crystal and the like).
∆: stability is slightly poor (precipitate is slightly formed or separation is slightly observed, or small cluster and crystals and bigger cluster are slightly formed).
X: stability is poor (precipitate is evidently formed or separated, and small cluster and crystals and bigger cluster are formed).

According to Inventive Examples 17 and 18, each of the creams of the invention is light in the sense of touch when used and not sticky after use, has superior rough skin improving effect and wrinkle improving effect, can provide the skin with moistness and liveliness, has excellent dinginess improving effect and is also excellent in periodical stability.

Inventive Examples 19 and 20

Skin cosmetics as water-in-oil type creams shown in Table 8 were prepared and their evaluation was Carried out by the same methods as in Inventive Examples 17 and 18. In this case, however, the 9 components shown in the aforementioned Table 4 were used as common adding components. The results are shown in Table 8.

According to Inventive Examples 19 and 20, each of the creams of the invention is light in the sense of touch when used and not sticky after use, has superior rough skin improving effect and wrinkle improving effect, can provide the skin with moistness and liveliness, has excellent dinginess improving effect and is also excellent in periodical stability.

Table 7

| | | | Inventive Examples | | | Comparative Examples | | | |
|---|---|---|---|---|---|---|---|---|---|
| | | | 11 | 12 | 13 | 5 | 6 | 7 | 8 |
| Compositions of skin cosmetics | | | | | | | | | |
| (A) | | Pecan seed hull extract A-1 | 0.1 | - | - | - | - | - | - |
| | | Pecan seed hull extract A-2 | - | 0.2 | 0.05 | - | 0.2 | - | - |
| (B) | | Retinol palmitate (note 1) | 0.1 | - | - | - | - | - | - |
| | | d-δ-Tocopherol (note 2) | - | - | 0. 05 | - | - | - | - |
| | | dl-α-Tocopherol acetate (note 3) | - | 0.3 | - | 0.3 | - | 0.3 | 0.3 |
| Others | | | | | | | | | |
| | | Green tea catechin (note 4) | - | - | - | - | - | 0.2 | - |
| | | Walnut seed hull extract A-3 | - | - | - | - | - | - | 0.2 |
| | | Glycerol | - | 5 | 2 | 5 | 5 | 5 | 5 |
| | | Dipropylene glycol | - | - | 3 | - | - | - | - |
| | | Polyethylene glycol 1540 | - | - | 2 | - | - | - | - |
| | | Polyoxyethylene sorbitan monooleate (20 E.O.) | 0.5 | 1 | 0.3 | 1 | 1 | 1 | 1 |
| | | Sodium pyrrolidonecarboxylate (50% aqueous solution) | - | - | 0.3 | - | - | - | - |
| | | Citric acid monohydrate | 0.1 | 0.1 | 0.03 | 0.1 | 0.1 | 0.1 | 0.1 |
| | | Oleyl alcohol | - | - | 0.05 | - | - | - | - |
| | | Perfume | - | - | 0.05 | - | - | - | - |
| | | Common additive components | 3.6 | | | 3.6 | | | |
| | | Purified water | balance | | | balance | | | |
| Performance | | | | | | | | | |
| | | The sense of touch when used | 1.9 | 1.9 | 1.8 | 1.8 | 1.8 | 1.9 | 1.7 |
| | | Stickiness after use | 2.0 | 1.8 | 1.8 | 1.6 | 1.7 | 1.8 | 1.7 |
| | | Rough skin improving affect | 1.8 | 1.8 | 1.8 | 1.1 | 0.8 | 1.0 | 1.3 |
| | | Wrinkle improving effect | 1.8 | 1.7 | 1.7 | 1.0 | 1.2 | 1.1 | 1.3 |
| | | Effect to provide the skin with moistness and liveliness | 1.9 | 1.8 | 1.8 | 1.2 | 1.5 | 1.2 | 1.2 |
| | | Dinginess improving effect | 1.7 | 1.8 | 1.8 | 1.3 | 1.4 | 1.2 | 1.3 |
| | | Periodical stability | O | O | O | O | O | X | Δ |

(Note 1) Retinal palmitate (Nippon Roche, vitamin A palmitate: 1,000,000 IU/g (0.55 g/g))

(Note 2) d-δ-Tocopherol (Eizai)

(Note 3) dl-α-Tocopherol acetate (Nippon Roche)

(Note 4) Green tea catechin (Taiyo Kagaku, SunPhenon 100)

Table 8

| | | | Inventive Examples | | | | | | |
|---|---|---|---|---|---|---|---|---|---|
| | | | 14 | 15 | 16 | 17 | 18 | 19 | 20 |
| Compositions of skin cosmetics | | | | | | | | | |
| (A) | Pecan seed hull extract A-1 | | 0.1 | - | - | 0.2 | - | 0.5 | - |
| | Pecan seed hull extract A-2 | | - | 0.3 | 0.03 | - | 0.5 | - | 0.3 |
| (B) | Retinol palmitate (note 1) | | 0.2 | - | 0.1 | - | - | 1 | - |
| | d-δ-Tocopherol (note 2) | | - | - | 0.1 | 1 | - | - | 0.3 |
| | dl-α-Tocopherol acetate (note 3) | | - | 0.5 | - | - | 0.1 | 0.5 | - |
| | 6-Palmitoyl-L-ascorbic acid (note 4) | | - | - | - | - | 0.1 | 2 | - |
| Others | | | | | | | | | |
| Glycerol | | | 2 | 2 | 2 | 2 | 2 | 2 | 3 |
| Dipropylene glycol | | | 2 | 3 | 3 | 3 | 3 | - | - |
| Polyethylene glycol 1540 | | | 2 | - | - | - | - | - | - |
| Polyethylene glycol monostearate (1540) | | | 0.5 | 0.5 | 0.5 | - | - | - | - |
| Polyethylene glycol monostearate (4000) | | | - | - | - | 1 | 1 | - | - |
| Polyoxyethylene sorbitan monostearate (20 E.O.) | | | - | - | - | 1 | 1 | - | - |
| Glycerol monooleate | | | - | - | - | - | - | 1.5 | 1.5 |
| Diglycerol monooleate | | | - | - | - | - | - | 0.2 | 0.2 |
| Glycerol monostearate | | | 1 | 1 | 1 | 2 | 2 | - | - |
| Cetanol | | | 2 | - | - | 3 | - | - | 3 |
| Behenyl alcohol | | | - | 1 | 1 | - | 3 | 2 | - |
| Decamethylcyclopentane siloxane | | | - | - | - | 3 | - | - | - |
| Perfume | | | - | - | 0.05 | - | - | - | - |
| Common additive components | | | 16.37 | | | 17.4 | | 19.9 | |
| Purified water | | | balance | | | balance | | balance | |
| Performance | | | | | | | | | |
| The sense of touch when used | | | 1.9 | 1.8 | 1.8 | 1.8 | 1.8 | 1.7 | 1.7 |
| Stickiness after use | | | 1.8 | 1.8 | 1.9 | 1.7 | 1.8 | 1.7 | 1.6 |
| Rough skin improving effect | | | 1.8 | 1.9 | 1.8 | 1.9 | 1.9 | 1.9 | 2.0 |
| Wrinkle improving effect | | | 1.9 | 1.7 | 1.9 | 1.8 | 1.8 | 2.0 | 1.9 |
| Effect to provide the skin with moistness and liveliness | | | 1.8 | 1.9 | 1.8 | 1.9 | 1.9 | 1.9 | 1.8 |
| Dinginess improving effect | | | 1.8 | 1.9 | 1.8 | 1.9 | 1.8 | 1.8 | 1.9 |
| Periodical stability | | | O | O | O | O | O | O | O |

(Note 1) Retinol palmitate (Nippon Roche, vitamin A palmitate: 1,000,000 IU/g (0.55 g/g))

(Note 2) d-δ-Tocopherol (Eizai)

(Note 3) dl-α-Tocopherol acetate (Nippon Roche)

(Note 4) Ascorbyl Palmitate (Nippon Roche)

Inventive Examples 21 to 24 and Comparative Examples 9 to 11

Cleansing compositions as cleansing lotions having the compositions shown in Table 9 were prepared and their performance was evaluated by the following methods. The results are shown in Table 9. In this case, the pecan seed hull extracts A-1 and A-2 produced in Production Examples 1 and 2 were used herein.

(1) Foaming property

An aqueous solution of each composition having a concentration of 5% by weight was prepared, and its foam heights just after charging at 25°C and 5 minutes thereafter were measured by the Ross-Miles method. A sample having 250 mm or more in the foam height just after charging and showing a foam keeping ratio of 90% or more calculated by the following formula was evaluated as a composition having good foaming property.

Foam keeping ratio = [(foam height after 5

minutes)/(foam height just after charging of each sample)]

x 100

(2) Creaminess of foams

Using a panel of 20 females (22 to 37 years old), creaminess of foams during face washing using 5 g of each composition was judged as follows, and by obtaining average value of the 20 members, an average value of 1.5 points or more was evaluated as a composition capable of producing creamy foams.
Two points: felt that the foams were creamy.
One point: felt that the foams were slightly rough.
Zero point: felt that the foams were rough.

(3) Sense of touch to the skin after face washing

Using a panel of 20 females (22 to 37 years old), the sense of touch after face washing using 5 g of each composition was judged as follows, and by obtaining average value of the 20 members, an average value of 1.5 points or more was evaluated as a composition having good sense of touch to the skin after face washing.
Two points: the skin was not strained but felt refreshing after face washing.
One point: the skin was slightly strained or felt slightly refreshing after face washing.
Zero point: the skin was strained or did not feel refreshing after face washing.

(4) Liveliness and moistness of the skin after face washing

Using a panel of 20 females (22 to 37 years old), the sense of touch after face washing using 5 g of each composition was judged as follows, and by obtaining average value of the 20 members, an average value of 1.5 points or more was evaluated as a composition capable of providing the skin with liveliness and moistness.
Two points: felt that the skin after face washing gained liveliness and moistness.
One point: felt that the skin after face washing was slightly poor in liveliness or slightly poor in moistness.
Zero point: felt that the skin after face washing became dry and rough having no liveliness and moistness.

(5) Periodical stability

Each of the compositions was sealed in a transparent glass container and stored at -5°C, 25°C or 45°C for 1 month, and its appearance was observed and evaluated by the following three steps.

O: stability is good (liquid and no changes in appearance under each temperature condition).
Δ: stability is slightly poor (a slight separation or a slight change of color is generated under any of the temperature conditions).
X: stability is poor (becomes a coagulated state or causes separation, or a change of color is considerable at any of the temperature conditions).

(6) Restorability

Each of the compositions was sealed in a transparent glass container, solidified at -20°C and then allowed to stand at room temperature for 6 hours to effect its restoration to the liquid state, and its appearance after repeating this process three times was observed and evaluated by the following three steps.

O: restorability is good (liquid and no changes in appearance).

Δ: restorability is slightly poor (viscosity is increased, or a slight separation or precipitation occurs).

X: restorability is poor (it coagulates or causes separation or precipitation).

(7) Odor removing effect

Using a panel of 20 females (22 to 37 years old), odors on fingers after soaking the fingers in 1% by weight ammonia aqueous solution for 1 minute, washing them using 5 g of each composition and then thoroughly rinsing them with water were judged as follows, and by obtaining average value of the 20 members, an average value of 1.5 points or more was evaluated as a composition having odor removing effect.

Two points: felt that there was no ammoniacal odor after washing.

One point: felt that there was slight ammoniacal odor after washing.

Zero point: felt that ammoniacal odor was evidently remained after washing.

Table 9

| | | Inventive Example | | | | Comparative Ex. | | |
|---|---|---|---|---|---|---|---|---|
| | | 21 | 22 | 23 | 24 | 9 | 10 | 11 |
| Composition (% by weight) of cleansing lotion | | | | | | | | |
| (A) | Pecan seed hull extract A-1 | 0.1 | - | 0.2 | 0.2 | 0.2 | - | - |
| | Pecan seed hull extract A-2 | - | 0.5 | - | 0.2 | - | - | - |
| (B) Fatty acid soap | Potassium laurate | 9 | 9 | 5 | 12 | 1 | 9 | 9 |
| | Potassium myristate | 5 | 4 | 4 | 6 | 3 | 5 | 5 |
| | Potassium palmitate | 2 | 2 | - | 3 | - | 2 | 2 |
| | Potassium oleate | 3 | 2 | 1 | 2 | - | 3 | 3 |
| | Potassium stearate | - | - | - | 0.5 | - | - | - |
| (B) Amphoteric surface active agent, Alkanolamide nonionic surface active agent | N-Cocoyl-N'-hydroxyethyl-N'-carboxymethylethylenediami ne sodium | | 2 | 3 | - | 3 | - | - |
| | Palm kernel oil fatty acid amidopropyldimethylaminoacetic acid betaine | - | 3 | - | - | - | - | - |
| | Monosodium lauryliminodiacetate | 3 | - | - | 1 | - | 3 | 3 |
| | Diethanolamide laurate | - | - | 5 | - | 5 | - | - |
| | Monoisopropanolamide laurate | - | - | - | 1 | - | - | - |
| Others | Diglycerol | - | - | - | 3 | - | - | - |
| | Methyl paraben | 0.2 | 0.2 | 0.2 | 0.2 | 0.2 | 0.2 | 0.2 |
| | Propyl paraben | 0.1 | 0.1 | 0.1 | 0.1 | 0.1 | 0.1 | 0.1 |
| | Catechin (note 1) | - | - | - | - | - | - | 0.1 |
| Purified water | | balance | | | | balance | | |
| Performance | | | | | | | | |
| Foam height just after sample charging (mm) | | 263 | 253 | 255 | 262 | 187 | 261 | 259 |
| Foam keeping ratio (%) | | 96 | 97 | 96 | 96 | 73 | 96 | 97 |
| Creaminess of foams | | 1.8 | 1.8 | 1.9 | 1.7 | 1.7 | 1.6 | 1.7 |
| Sense of touch to the skin after face washing | | 1.8 | 1.9 | 1.8 | 1.8 | 1.3 | 0.7 | 0.9 |

(note 1) "SunPhenon 100" manufactured by Taiyo Kagaku

Table 9   (continued)

| | Inventive Example | | | | Comparative Ex. | | |
|---|---|---|---|---|---|---|---|
| Performance | | | | | | | |
| Liveliness and moistness of the skin after face washing | 1.8 | 1.8 | 1.9 | 1.8 | 1.7 | 0.9 | 0.7 |
| Periodical stability | O | O | O | O | O | O | O |
| Restorability | O | O | O | O | O | O | O |
| Odor removing effect | 1.8 | 1.9 | 1.9 | 1.8 | 1.8 | 0.3 | 1.4 |

According to Table 9, each of the cleansing lotions as the cleansing compositions (Inventive Examples 21 to 24) of the invention showed excellent foaming property, excellent periodical stability and restorability from low temperature storage, excellent creaminess of foams and good sense of touch after washing, provided the skin with liveliness and moistness after washing, and was also excellent in odor removing effect. On the other hand, sufficient performance was not obtained in Comparative Examples 9 to 11. That is, the foaming property became extremely poor in Comparative Example 9, because the component (B) was formulated in an amount smaller than the range of the invention, and the sense of touch to the skin after washing became poor and the effect to provide the skin with liveliness and moistness after washing and the odor removing effect were also worsened in Comparative Examples 10 and 11, because the component (A) was not formulated or formulated by changing it to the green tea catechin as a plant polyphenol.

Inventive Examples 25 to 29 and Comparative Examples 12 and 13

Cleansing compositions as cleansing creams having the compositions shown in Table 10 were prepared, and their (1) foaming property, (2) creaminess of foams, (3) sense of touch to the skin after face washing, (4) liveliness and moistness of the skin after face washing and (7) odor removing effect were evaluated by the same methods as in Inventive Examples 21 to 24, and the (5) periodical stability and (6) restorability were evaluated by the following methods. The results are shown in Table 10. In this case, the pecan seed hull extracts A-1 and A-2 produced in Production Examples 1 and 2 were used herein.

(5) Periodical stability

Each of the compositions was sealed in a transparent glass container and stored at -5°C, 25°C or 40°C for 1 month, and its appearance was observed and evaluated by the following three steps.

O: stability is good (no changes in appearance under each temperature condition).
Δ: stability is slightly poor (a slight separation or a slight change of color is generated under any of the temperature conditions).
X: stability is poor (separation or a change of color is considerable at any of the temperature conditions, or cluster is formed or the ground becomes soppy).

(6) Restorability

Each of the compositions was sealed in a transparent glass container, solidified at -20°C and then allowed to stand at room temperature for 6 hours to effect its restoration to the creamy state. After repeating this process three times, its appearance was observed and evaluated by the following three steps.

O: restorability is good (no changes in appearance).
Δ: restorability is slightly poor (slightly causes separation, or slightly forms cluster).
X: restorability is poor (causes separation, or cluster is formed or the ground becomes soppy).

Table 10

| | | Inventive Example | | | | | Comp. Ex. | |
|---|---|---|---|---|---|---|---|---|
| | | 25 | 26 | 27 | 28 | 29 | 12 | 13 |
| Composition (% by weight) of cleansing cream | | | | | | | | |
| (A) | Pecan seed hull extract A-1 | 0.1 | - | - | 0.05 | - | - | - |
| | Pecan seed hull extract A-2 | - | 0.3 | 0.05 | - | 0,5 | - | - |
| (B) Fatty acid soap | Lauric acid | 2 | 5 | 3 | - | - | 2 | 5 |
| | Myristic acid | 22 | 15 | 20 | 25 | 25 | 22 | 15 |
| | Palmitic acid | 3 | 5 | 3 | 5 | 4 | 3 | 5 |
| | Oleic acid | - | - | 2 | - | 2 | - | - |
| | Stearic acid | 8 | 5 | 5 | 4 | 6 | 8 | 5 |
| | Potassium hydroxide 48 wt % aqueous solution | 12.8 | 10.6 | 12.1 | 12.5 | 14.2 | 12.8 | 10.6 |
| | Sodium hydroxide 48 wt % aqueous solution | 0.5 | 0.9 | 0.5 | 0.5 | - | 0.5 | 0.9 |
| (B) Amphoteric surface active agent, Alkanolami de nonionic surface active agent | Palm kernel oil fatty acid amidopropyldimethylaminoacetic acid betaine | - | 3 | 2 | - | - | - | 3 |
| | Monosodium lauryliminodiacetate | 2 | - | 2 | 3 | 2 | 2 | - |
| | Diethanolamide laurate | 1 | - | - | 1 | - | 1 | - |
| | Monoisopropanolamide laurate | - | 1 | - | 1 | - | - | 1 |
| Others | Coconut oil fatty acid amide ether sodium sulfate | 1 | 2 | - | - | - | 1 | 2 |
| | Ethylene glycol distearate | 2.5 | 2.5 | 2.5 | 2.5 | 2.5 | 2.5 | 2.5 |
| | Glycerol | 7 | 10 | 8 | 6 | 10 | 7 | 10 |
| | 1,3-Butylene glycol | 8 | 10 | 10 | 9 | 8 | 8 | 10 |
| | Methyl paraben | 0.2 | 0.2 | 0.2 | 0.2 | 0.2 | 0.2 | 0.2 |
| | Propyl paraben | 0.1 | 0.1 | 0.1 | 0.1 | 0.1 | 0.1 | 0.1 |
| Purified water | | balance | | | | | balance | |
| performance | | | | | | | | |
| Foam height just after sample charging (mm) | | 254 | 257 | 258 | 253 | 255 | 251 | 258 |
| Foam keeping ratio (%) | | 97 | 97 | 96 | 97 | 96 | 97 | 96 |
| Creaminess of foams | | 1.8 | 1.8 | 1.8 | 1.7 | 1.8 | 1.6 | 1.7 |
| Sense of touch to the skin after face washing | | 1.8 | 1.7 | 1.9 | 1.7 | 1.8 | 1.1 | 0,9 |
| Liveliness and moistness of the skin after face washing | | 1.7 | 1.9 | 1.8 | 1.7 | 1.9 | 1.3 | 1.2 |
| Periodical stability | | O | O | O | O | O | O | O |
| Restorability | | O | O | O | O | O | O | O |
| Odor removing effect | | 1.8 | 1.9 | 1.7 | 1.7 | 1.8 | 0.2 | 0.5 |

According to Table 10, each of the cleansing cream as the cleansing compositions (Inventive Examples 25 to 29) of the invention showed excellent foaming property, excellent periodical stability and restorability from low temperature storage, excellent creaminess of foams and good sense of touch after washing, provided the skin with liveliness and moistness after washing, and was also excellent in odor removing effect. On the other hand, sufficient performance was not obtained in Comparative Examples 12 and 13. That is, the sense of touch to the skin after washing became poor and the effect to provide the skin with liveliness and moistness after washing and the odor removing effect were also worsened, because the component (A) was not formulated.

Inventive Examples 30 to 33 and Comparative Examples 14 and 15

Cleansing compositions as body shampoos having the compositions shown in Table 11 were prepared, and their (1) foaming property, (5) periodical stability, (6) restorability and (7) odor removing effect were evaluated by the methods described in Inventive Examples 21 to 24, and their (2) creaminess of foams, (3) sense of touch to the skin after washing and (4) liveliness and moistness of the skin after washing were evaluated by the following methods. The results are shown in Table 11. In this case, the pecan seed hull extracts A-1 and A-2 produced in Production Examples 1 and 2 were used herein. Also, the walnut seed hull extract A-3 produced in Comparative Production Example 1 was used herein.

(2) Creaminess of foams

Using a panel of 20 females (22 to 37 years old), creaminess of foams during washing of fingers and forearm regions using 5 g of each composition put on a bathing sponge was judged as follows, and by obtaining average value of the 20 members, an average value of 1.5 points or more was evaluated as a composition capable of producing creamy foams.
Two points: felt that the foams were creamy.
One point: felt that the foams were slightly rough .
Zero point: felt that the foams were rough.

(3) Sense of touch to the skin after washing

Using a panel of 20 females (22 to 37 years old), the sense of touch after washing of fingers and forearm regions using 5 g of each composition put on a bathing sponge was judged as follows, and by obtaining average value of the 20 members, an average value of 1.5 points or more was evaluated as a composition having good sense of touch to the skin after washing.
Two points: the skin was not strained but felt refreshing after washing.
One point: the skin was slightly strained or felt Slightly refreshing after washing.
Zero point: the skin was strained or did not feel refreshing after washing.

(4) Liveliness and moistness of the skin after washing

Using a panel of 20 females (22 to 37 years old), the sense of touch after washing of fingers and forearm regions using 5 g of each composition put on a bathing sponge was judged as follows, and by obtaining average value of the 20 members, an average value of 1.5 points or more was evaluated as a composition capable of providing the skin with liveliness and moistness,
**[0156]** Two points: felt that the skin after washing gained liveliness and moistness.
One point: felt that the skin after washing was slightly poor in liveliness or slightly poor in moistness.
Zero point: felt that the skin after washing became dry and rough having no liveliness and moistness.

Table 11

| | | Inventive Example | | | | Comp. Ex. | |
|---|---|---|---|---|---|---|---|
| | | 30 | 31 | 32 | 33 | 14 | 15 |
| Composition (% by weight) of body shampoo | | | | | | | |
| (A) | Pecan seed hull extract A-1 | 0.2 | - | - | - | - | - |
| | Pecan seed hull extract A-2 | - | 0.01 | 0.5 | 0.1 | - | - |

Table 11   (continued)

| | | Inventive Example | | | | Comp. Ex. | |
|---|---|---|---|---|---|---|---|
| | | 30 | 31 | 32 | 33 | 14 | 15 |
| Composition (% by weight) of body shampoo | | | | | | | |
| (B) Fatty acid soap | Potassium laurate | 12 | 10 | 10 | 8 | 10 | 10 |
| | Potassium myristate | 2 | 4 | 5 | 2 | 4 | 5 |
| | Potassium palmitate | 2 | 2 | 2 | 3 | 2 | 2 |
| | Potassium oleate | 2 | 1 | - | 4 | 1 | - |
| | Potassium stearate | - | 0.5 | 1 | - | 0.5 | 1 |
| (B) Amphoteric surface active agent, Alkanolami de nonionic surface active agent | N-Cocoyl-N'-hydroxyethyl-N'-carboxymethylethylenediamine sodium | - | - | 3 | 3 | - | 3 |
| | Palm kernel oil fatty acid amidopropyldimethylaminoacetic acid betaine | - | 3 | - | 1 | 3 | - |
| | Diethanolamide laurate | - | 1 | 2 | - | 1 | 2 |
| Others | Walnut seed bull extract A-3 | - | - | - | - | - | 0.5 |
| | Coconut oil fatty acid amide ether sodium sulfate | 1 | 1 | 3 | - | 2 | 3 |
| | N-Cocoyl-N-methyltaurine sodium | - | 2 | - | 2 | - | - |
| | Hydroxyethyl cellulose | 0.5 | - | - | - | 0.5 | - |
| | EDTA-4Na | 0.2 | 0.2 | 0.2 | 0.2 | 0.2 | 0.2 |
| | Ethylene glycol distearate | 2 | - | - | 2 | 2 | - |
| | Methyl paraben | 0.2 | 0.2 | 0.2 | 0.2 | 0.2 | 0.2 |
| | Propyl paraben | 0.1 | 0.1 | 0.1 | 0.1 | 0.1 | 0.1 |
| | perfume | - | - | - | 0.1 | 0.1 | - |
| Purified water | | balance | | | | balance | |
| Performance | | | | | | | |
| Foam height just after sample charging (mm) | | 263 | 259 | 260 | 257 | 265 | 260 |
| Foam keeping ratio (%) | | 97 | 97 | 95 | 96 | 95 | 96 |
| Creaminess of foams | | 1.5 | 1.8 | 1.9 | 1.9 | 1.7 | 1.9 |
| Sense of touch to the skin after washing | | 1.9 | 18 | 1.8 | 1.7 | 1.1 | 1.3 |
| Liveliness and moistness of the skin after face washing | | 1.8 | 1.7 | 1.8 | 1.9 | 1.0 | 1.1 |
| Periodical stability | | O | O | O | O | O | O |
| Restorability | | O | O | O | O | O | O |
| Odor removing effect | | 1.9 | 1.7 | 1.9 | 1.8 | 0.8 | 1.0 |

According to Table 11, each of the liquid body shampoos as the cleansing compositions (Inventive Examples 30 to 33) of the invention showed excellent foaming property, excellent periodical stability and restorability from low temperature storage, excellent creaminess of foams and good sense of touch after washing, provided the skin with liveliness and moistness after washing, and was also excellent in odor removing effect. On the other hand, sufficient performance was not obtained in Comparative Examples 14 and 15. That is, the sense of touch to the skin after washing and the liveliness and moistness of the skin after washing became poor and the odor removing effect was also worsened in Comparative Examples 14 and 15, because the pecan seed hull extract was not formulated or the walnut seed hull extract was formulated instead thereof.

Inventive Examples 34 to 36

Cleansing compositions as liquid hand soap having the compositions shown in Table 12 were prepared and their (1) foaming property, (5) periodical stability and (6) restorability were evaluated by the methods described in Inventive Examples 21 to 24, and their (2) creaminess of foams, (3) sense of touch to the skin after washing, (4) liveliness and moistness of the skin after washing and (7) odor removing effect were evaluated by the following methods. The results are shown in Table 12. In this case, the pecan seed hull extracts A-1 and A-2 produced in Production Examples 1 and 2 were used herein.

(2) Creaminess of foams

Using a panel of 20 females (22 to 37 years old), creaminess of foams during washing of fingers using 5 g of each composition was judged as follows, and by obtaining average value of the 20 members, an average value of 1.5 points or more was evaluated as a composition capable of producing creamy foams.
Two points: felt that the foams were creamy.
One point: felt that the foams were slightly rough.
Zero point: felt that the foams were rough.

(3) Sense of touch to the skin after washing

Using a panel of 20 females (22 to 37 years old), the sense of touch after washing of fingers using 5 g of each composition was judged as follows, and by obtaining average value of the 20 members, an average value of 1.5 points or more was evaluated as a composition having good sense of touch to the skin after washing.
Two points: the skin was not strained but felt refreshing after washing.
One point: the skin was slightly strained or felt slightly refreshing after washing.
Zero point: the skin was strained or did not feel refreshing after washing.

(4) Liveliness and moistness of the skin after washing

Using a panel of 20 females (22 to 37 years old), the sense of touch after washing of fingers using 5 g of each composition was judged as follows, and by obtaining average value of the 20 members, an average value of 1.5 points or more was evaluated as a composition capable of providing the skin with liveliness and moistness after washing.
Two points: felt that the skin after washing gained liveliness and moistness.
One point: felt that the skin after washing was slightly poor in liveliness or slightly poor in moistness.
Zero point: felt that the skin after washing became dry and rough having no liveliness and moistness.

(7) odor removing effect

Using a panel of 10 females (25 to 37 years old), odors remained on fingers after treating a raw sardine (about 20 cm) with a kitchen knife and then washing the fingers using 5 g of each composition were judged as follows, and by obtaining average value of the 10 members, an average value of 1.5 points or more was evaluated as a composition having excellent odor removing effect.
Two points: felt that there was no fishy smell after washing.
One point: felt that there was slight fishy smell after washing.
Zero point: felt that fishy smell was evidently remained after washing.

Table 12

|  |  | Inventive Ex. | | |
|---|---|---|---|---|
|  |  | 34 | 35 | 36 |
| Composition (% by weight) of liquid hand soap | | | | |
| (A) | Pecan seed hull extract A-1 | 0.7 | - | - |
|  | Pecan seed hull extract A-2 | - | 0.5 | 0.5 |

Table 12   (continued)

| | | Inventive Ex. | | |
|---|---|---|---|---|
| | | 34 | 35 | 36 |
| Composition (% by weight) of liquid hand soap | | | | |
| (B) Fatty acid soap | Potassium laurate | 13 | 12 | 10 |
| | Potassium myristate | 2 | 3 | 3 |
| | Potassium palmitate | 2 | 1 | 3 |
| | Potassium oleate | - | - | 2 |
| | Potassium stearate | - | 0.5 | - |
| (B) Amphoteric surface active agent, Alkanolamide nonionic surface active agent | N-Cocoyl-N'-hydroxyethyl-N'-carboxymethylethylenediamine sodium | - | 2 | - |
| | Palm kernel oil fatty acid amidopropyldimethylaminoacetic acid betaine | 1 | - | 2 |
| | Diethanolamide laurate | - | 2 | - |
| Others | Coconut oil fatty acid amide ether sodium sulfate | 2 | 2 | - |
| | N-Cocoyl-N-methyltaurine sodium | - | - | 2 |
| | Hydroxyethyl cellulose | 0.5 | - | - |
| | EDTA-4Na | 0.2 | 0.2 | 0.2 |
| | Propylene glycol | - | 3 | 5 |
| | Ethylene glycol distearate | 2 - | - | 2 |
| | Methyl paraben | 0.2 | 0.2 | 0.2 |
| | Propyl paraben | 0.1 | 0.1 | 0.1 |
| | perfume | 0.1 | - | 0.1 |
| Purified water | | balance | | |
| Performance | | | | |
| Foam height just after sample charging (mm) | | 263 | 261 | 256 |
| Foam keeping ratio (%) | | 96 | 96 | 97 |
| Creaminess of foams | | 1.8 | 1.9 | 1.7 |
| Sense of touch to the skin after washing | | 1.9 | 1.8 | 1.9 |
| Liveliness and moistness of the skin after washing | | 1.7 | 1.9 | 1.8 |
| Periodical stability | | O | O | O |
| Restorability | | O | O | O |
| Odor removing effect | | 1.7 | 1.8 | 1.9 |

According to Table 12, each of the liquid hand soaps as the cleansing compositions (Inventive Examples 34 to 36) of the invention showed excellent foaming property, excellent periodical stability and restorability from low temperature storage, excellent creaminess of foams and good sense of touch after washing, provided the skin with liveliness and moistness after washing, and was also excellent in odor removing effect.

As has been described in the foregoing in detail, skin cosmetics which are excellent in moistness and persistency of moisture-keeping effect, show light sense of touch when used and have excellent periodical stability can be provided by the invention. In addition, it can provide skin cosmetics which do not cause stickiness after their use, are excellent in rough skin improving and preventing effects, wrinkle preventing and improving effects, dinginess preventing and improving effects and the like, and also have such effects that they can keep the skin young by providing the skin with smoothness and liveliness.

Also, in the case of skin cleansing compositions, they exert such effects that they can provide the skin with liveliness and moistness after washing and can effectively remove fishy and ammonia smells or the like filthy smell adhered to the hand, the skin and the like.

While the invention has been described in detail and with reference to specific embodiments thereof, it will be apparent to one skilled in the art that various changes and modifications can be made therein without departing from the scope thereof.

This application is based on Japanese patent applications No. 2002-348360 filed November 29, 2002, No. 2002-370231 filed December 20, 2002, and No. 2002-370238 filed December 20, 2002, the entire contents thereof being hereby incorporated by reference.

**Claims**

1. A skin cosmetic which comprises (A) at least one substance selected from seed extracts and seed hull extracts of a plant belonging to the genus *Carya* of the family *Juglandaceae* in an amount of from 0.00005 to 2% by weight as solid matter, and (B) an additive agent.

2. The skin cosmetic according to claim 1, wherein the additive agent (B) is an anti-inflammatory agent,

3. The skin cosmetic according to claim 2, wherein the anti-inflammatory agent is at least one compound selected from: glycyrrhizic acid and/or a derivative thereof, glycyrrhetinic acid and/or a derivative thereof, azulene and/or a derivative thereof, and allantoin.

4. The skin cosmetic according to claim 1, wherein the additive agent (B) is an oil soluble antioxidant.

5. The skin cosmetic according to claim 4, wherein the oil soluble antioxidant is at least one compound selected from vitamin E and/or a derivative thereof, vitamin A and/or a derivative thereof, and a derivative of vitamin C.

6. The skin cosmetic according to claim 1, wherein the additive agent (B) is a fatty acid soap having from B to 20 carbon atoms.

7. The skin cosmetic according to claim 1, wherein the additive agent (B) is a fatty acid soap having from 8 to 20 carbon atoms, and an amphoteric surface active agent and/or an alkanolamide nonionic surface active agent.

8. The skin cosmetic according to claim 6, wherein the skin cosmetic is a skin cleansing composition.

9. The skin cosmetic according to claim 7, wherein the skin cosmetic is a skin cleansing composition.

# EP 1 428 523 A1

## EUROPEAN SEARCH REPORT

**European Patent Office**

**Application Number**

EP 03 29 2955

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X,D | JP 2000 072686 A (TAKASAGO INTERNATL CORP) 7 March 2000 (2000-03-07) | 1,6-9 | A61K7/48 |
| Y | * examples 14-18 * | 2-5 | |
| Y,D | US 5 962 000 A (OKIHIKO SAKAMOTO ET AL) 5 October 1999 (1999-10-05) * claims * | 2-5 | |
| Y,D | US 5 686 086 A (OKIHIKO SAKAMOTO ET AL) 11 November 1997 (1997-11-11) * claims * | 2-5 | |
| A | WO 00/32163 A (GATTEFOSSE ETS SA ; LAFORET JEAN PIERRE (FR)) 8 June 2000 (2000-06-08) * claims; examples * | 1-9 | |
| A | DATABASE WPI Section Ch, Week 199440 Derwent Publications Ltd., London, GB; Class D21, AN 1994-322446 XP002274687 & JP 06 248297 A (LION CORP) 6 September 1994 (1994-09-06) * abstract * | 1-9 | **TECHNICAL FIELDS SEARCHED (Int.Cl.7)** <br><br> A61K |
| A | DATABASE WPI Section Ch, Week 199519 Derwent Publications Ltd., London, GB; Class B04, AN 1995-144677 XP002274688 & JP 07 069853 A (LION CORP) 14 March 1995 (1995-03-14) * abstract * | 1-9 | |

The present search report has been drawn up for all claims

| Place of search | Date of completion of the search | Examiner |
|---|---|---|
| Berlin | 24 March 2004 | Boeker, R |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

26

## ANNEX TO THE EUROPEAN SEARCH REPORT
## ON EUROPEAN PATENT APPLICATION NO.

EP 03 29 2955

This annex lists the patent family members relating to the patent documents cited in the above-mentioned European search report.
The members are as contained in the European Patent Office EDP file on
The European Patent Office is in no way liable for these particulars which are merely given for the purpose of information.

24-03-2004

| Patent document cited in search report | | Publication date | Patent family member(s) | | Publication date |
|---|---|---|---|---|---|
| JP 2000072686 | A | 07-03-2000 | NONE | | |
| US 5962000 | A | 05-10-1999 | JP | 3018130 B2 | 13-03-2000 |
| | | | JP | 6024956 A | 01-02-1994 |
| | | | JP | 2906198 B2 | 14-06-1999 |
| | | | JP | 6024957 A | 01-02-1994 |
| | | | JP | 2720256 B2 | 04-03-1998 |
| | | | JP | 6032728 A | 08-02-1994 |
| | | | AT | 235883 T | 15-04-2003 |
| | | | DE | 69332818 D1 | 08-05-2003 |
| | | | DE | 69332818 T2 | 06-11-2003 |
| | | | EP | 0610511 A1 | 17-08-1994 |
| | | | ES | 2191664 T3 | 16-09-2003 |
| | | | WO | 9401083 A1 | 20-01-1994 |
| | | | US | 5686086 A | 11-11-1997 |
| US 5686086 | A | 11-11-1997 | JP | 3018130 B2 | 13-03-2000 |
| | | | JP | 6024956 A | 01-02-1994 |
| | | | JP | 2906198 B2 | 14-06-1999 |
| | | | JP | 6024957 A | 01-02-1994 |
| | | | JP | 2720256 B2 | 04-03-1998 |
| | | | JP | 6032728 A | 08-02-1994 |
| | | | AT | 235883 T | 15-04-2003 |
| | | | DE | 69332818 D1 | 08-05-2003 |
| | | | DE | 69332818 T2 | 06-11-2003 |
| | | | EP | 0610511 A1 | 17-08-1994 |
| | | | ES | 2191664 T3 | 16-09-2003 |
| | | | WO | 9401083 A1 | 20-01-1994 |
| | | | US | 5962000 A | 05-10-1999 |
| WO 0032163 | A | 08-06-2000 | FR | 2786394 A1 | 02-06-2000 |
| | | | AT | 233082 T | 15-03-2003 |
| | | | DE | 69905603 D1 | 03-04-2003 |
| | | | DE | 69905603 T2 | 18-09-2003 |
| | | | DK | 1131048 T3 | 31-03-2003 |
| | | | EP | 1131048 A1 | 12-09-2001 |
| | | | ES | 2189543 T3 | 01-07-2003 |
| | | | WO | 0032163 A1 | 08-06-2000 |
| | | | US | 6395261 B1 | 28-05-2002 |
| JP 6248297 | A | 06-09-1994 | NONE | | |
| JP 7069853 | A | 14-03-1995 | NONE | | |

EPO FORM P0459

For more details about this annex : see Official Journal of the European Patent Office, No. 12/82